# EUROPEAN PATENT APPLICATION

(11) **EP 1 063 298 A2**
(43) Date of publication of application: **27.12.2000**
(21) Application number: 00304708.1
(22) Date of filing: 02.06.2000
(51) Int. Cl.: C12N 15/85, C12N 5/10, A01K 67/027, C07K 14/47, C07K 14/705, A61K 49/00, G01N 33/50

(54) **Transgenic Animal Model for Alzheimer Disease**

(30) Priority: 04.06.1999 GB 9913067; 04.06.1999 US 137424 P
(71) Applicant: GLAXO GROUP LIMITED, Greenford, Middlesex UB6 ONN (GB)
(72) Inventor: Richardson, Jill Caroline, Glaxo Wellcome plc, Stevenage, Hertfordshire SG1 2NY (GB); Rupniak, Herman Thomas Rene, Glaxo Wellcome plc, Stevenage, Hertfordshire SG1 2NY (GB)
(74) Representative: Woods, Geoffrey Corlett

(57) **Abstract**

A recombinant DNA construct comprises a coding sequence which encodes the human APP695 isoform containing only the mutations K670N and M671L and a brain-specific Thy-1 regulatory sequence which is operably linked to the coding sequence. The construct is useful in generating transgenic non-human animals and cells which can themselves be used to screen for potential therapeutic agents for neurodegenerative diseases such as Alzheimer's Disease.

## Description

### Field of Invention

The present invention relates to an animal model of the amyloidosis observed in the Alzheimer brain, and represents a tractable system in which to study the consequences of amyloid accumulation in the central nervous system (CNS). This animal model also represents a system for screening potential drugs for the treatment of Alzheimer's disease and other degenerative brain conditions such as head injury. The invention also relates to associated polynucleotide constructs, vectors, cells and methods for testing and screening such potential drugs.

### Background of Invention

Alzheimer's Disease (AD) is characterised by two principal hallmarks of pathology which are amyloid plaques and neurofibrillary tangles composed of aggregated β-amyloid peptide (βA₄: a 4kD peptide of 39-43 amino acids) and hyperphosphorylated tau respectively. βA₄ peptide is derived by proteolytic cleavage of amyloid precursor protein (APP) by the proteases, β- and γ-secretases. APP can also be cleaved by α-secretase within the region of APP which constitutes βA₄. Consequently, the α-secretase processing pathway precludes the formation of βA₄.

The three most abundant isoforms of APP are APP695, APP751 and APP770. These isoforms of APP are produced as a result of alternative splicing. The APP751 and APP770 isoforms contain the whole sequence of APP695 plus one or two additional domains respectively in a region of the APP protein distinct from the site of the βA₄ domain. It is currently unknown how each of the different isoforms contributes to the amyloid plaque pathology of AD. By convention, the amino acid positions of all forms of APP are referenced by the equivalent positions in the APP770 isoform. Unless otherwise specified, all references herein to APP and to the APP gene refer to human APP and to the human APP gene respectively.

In early onset familial AD genetically linked to APP and Presenilin mutations, the underlying cause of the disease has been postulated to be altered APP processing resulting in an increase in amyloidogenic processing of this protein and resultant amyloid pathology in the brain. Three mutations which cosegregate with certain early onset forms of familial AD have been identified in the APP gene. These mutations occur at, or close to, the sites of amyloidogenic processing. Two such mutations are K670N and M671L, which are together known as the Swedish mutation. The Swedish mutation has been shown to increase the production of total βA₄ four to five fold compared to wild-type APP.

The pathological signs characteristic of Alzheimers Disease do not occur spontaneously in rodents. Transgenic technology has been used to try to reproduce AD pathology in the mouse brain (WO 95/11968, WO 96/40895 and WO 98/03644). High over-expression of human APP containing familial AD mutations has previously been demonstrated to result in significant amyloid plaque pathology in transgenic mice. However, no such model provides a convincing correlation between increasing amyloid burden and development of cognitive deficits. In addition no models have been successful in reproducing other aspects of AD pathology such as neurofibrillary tangles and significant neuronal loss.

### Summary of Invention

We have generated several different models of AD in transgenic mice. We have found that use of a specific neuronal promoter with a specific APP isoform containing specific mutations results in transgenic mice which not only develop amyloid pathology but, for the first time, also show convincing age-related cognitive deficits that correlate to levels of βA₄ in the brain. Such mice allow inhibitors of amyloid production to be tested *in vivo* employing a functional readout (cognitive behaviour) as the endpoint. In addition to testing the activity of these compounds in the relevant tissue (i.e. the brain) this model allows testing of the hypothesis that amyloid burden is responsible for the cognitive changes observed in the ageing AD brain. Accordingly the invention provides:
- a recombinant DNA construct comprising a coding sequence which encodes the human APP695 isoform containing only the mutations K670N and M671L (the Swedish mutation) and a brain-specific Thy-1 regulatory sequence which is operably linked to the coding sequence;
- a transgenic non-human animal whose genome incorporates DNA comprising a coding sequence which encodes the human APP695 isoform containing only the mutations K670N and M671L and a brain-specific Thy-1 regulatory sequence which is operably linked to the coding sequence, the said mutated human APP695 isoform being expressed in brain cells of the animal in an amount at least five times greater than the amount of endogenous APP that is expressed therein;
- a transgenic non-human animal cell whose genome incorporates DNA comprising a coding sequence which encodes the human APP695 isoform containing only the mutations K670N and M671L and a brain-specific Thy-1 regulatory sequence which is operably linked to the coding sequence, the said mutated human APP695 isoform being expressed in the said cell in an amount at least five times greater than the amount of endogenous APP that is expressed therein;
- a method of producing a transgenic non-human animal, which method comprises:
   (a) introducing a using construct according to the invention into a cell or embryo; and
   (b) generating said animal from said cell or embryo.
- a method of screening agents for use in the treatment of a degenerative brain condition, which method comprises screening the said agents using a transgenic non-human animal or cell according to the invention;
- a kit for screening agents for use in the treatment of a degenerative brain condition, which kit comprises a transgenic non-human animal cell of the invention and a means for determining whether a test agent inhibits cellular changes associated with mutated APP expression in said cell;
- an agent identified using a screening method or kit according to the invention;
- an agent of the invention for use in the treatment of a degenerative brain condition;
- use of an agent of the invention in the manufacture of a medicament for the treatment of Alzheimer's disease.
- a pharmaceutical composition suitable for use in the treatment of a degenerative brain condition, which composition comprises a pharmaceutically acceptable carrier or diluent and, as active ingredient, an agent identified using a screening method or kit according to the invention; and
- a transgenic non-human animal that exhibits increasing amyloid burden and associated correlative cognitive decline in an age-related manner.

### Brief Description of Drawings

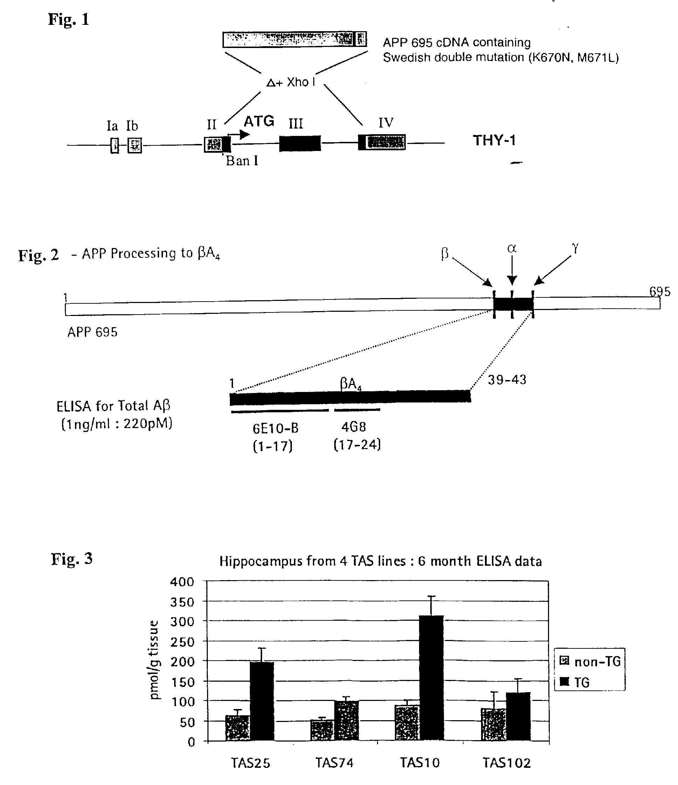

Figure 1 shows the Thy-1-APP transgene constructed by inserting the 695 isoform of human APP cDNA (APP695) harbouring the Swedish familial mutation into a vector containing the murine Thy-1 gene. The transgene was used to generate TAS lines of transgenic mice over-expressing APP695 harbouring the Swedish mutation under the control of the murine Thy-1 promoter.

Figure 2 illustrates how APP695 is processed to produce βA₄ and indicates the regions of βA₄ that are recognised by 4G8 and 6E10 antibodies.

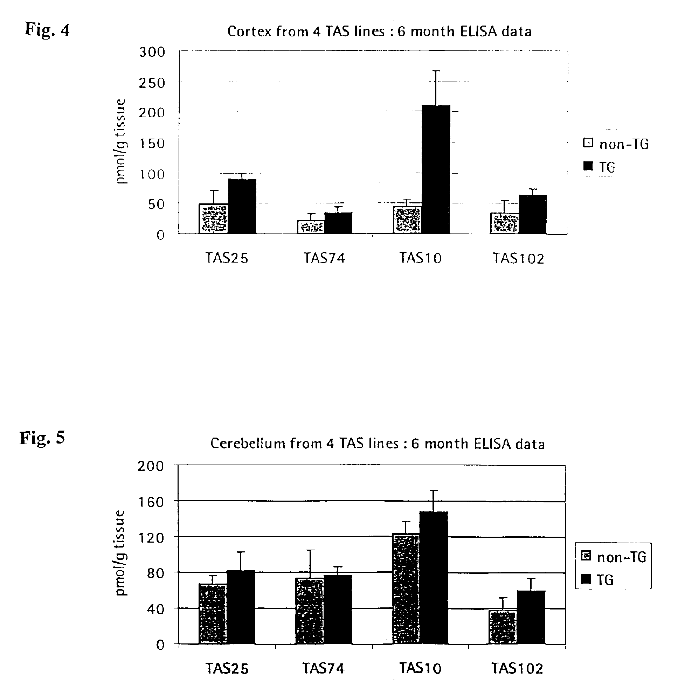

Figures 3, 4 and 5 show the results of sandwich ELISA assays carried out using 4G8 capture and biotinylated 6E10 reporter antibodies on extracts of hippocampal, cortical and cerebellar tissues respectively. Samples were tested in triplicate and absorbances which fell within the range of a βA₄ 1-40 standard curve (1-20ng/ml) were converted to picomoles and expressed as mean pmol/g wet tissue for each TAS line, either non-transgenic (non-TG) or transgenic (TG).

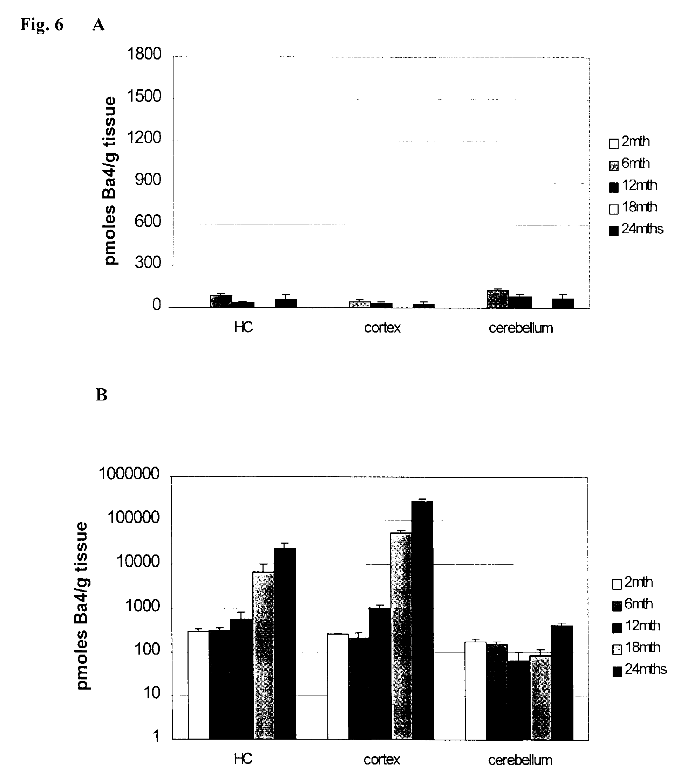

Figure 6 shows the results of sandwich ELISA assays carried out using 4G8 capture and biotinylated 6E10 reporter antibodies on extracts of hippocampal, cortical and cerebellar tissues from non-transgenic (Fig. 6a) or transgenic TAS 10 mice (Fig. 6b). Total βA₄ values expressed in pmol/g wet tissue (±SEM) are an average from 6 mice from each group.

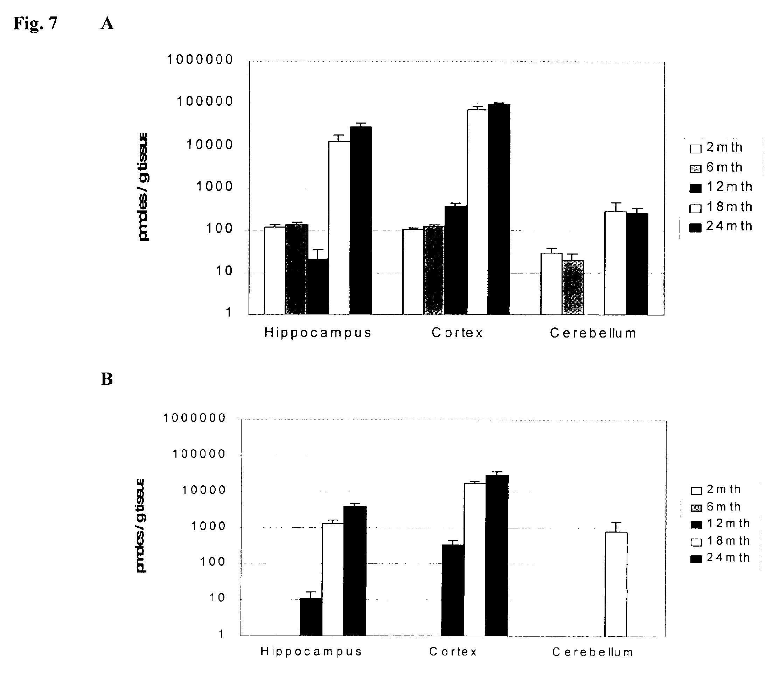

Figure 7 shows the results of sandwich ELISA assays carried out using 4G8 capture and biotinylated 6E10 reporter antibodies on extracts of hippocampal, cortical and cerebellar tissues respectively from transgenic TAS10 mice. βA₄ 1-40 (Fig. 7a) and βA₄ 1-42 (Fig. 7b) values expressed in pmol/g wet tissue (±SEM) are mean valves for 6 mice from each group.

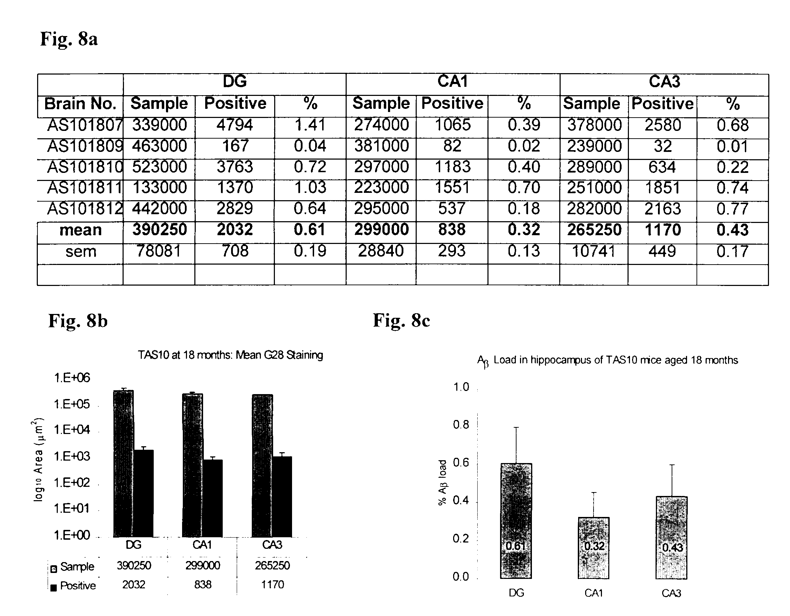

Figure 8 shows the results of quantitative image analysis of the amyloid burden in the hippocampus of TAS 10 transgenic mice at 18 months. Figure 8a is a summary of the raw data showing the % of the sample area occupied by plaques in the dentate gyrus (DG), CA1 and CA3 regions of the hippocampus. Six sections per brain were examined. Figure 8b shows the mean area stained with an antisera against the N-terminus (amino acids 1 to 17) of βA₄ for the DG, CA1 and CA3 regions of the hippocampus. Figure 8c shows the βA₄ load expressed as a % of the total sample area in each of the three brain regions studied. Age-matched wild-type littermate controls lacked amyloid plaques in all regions studied.

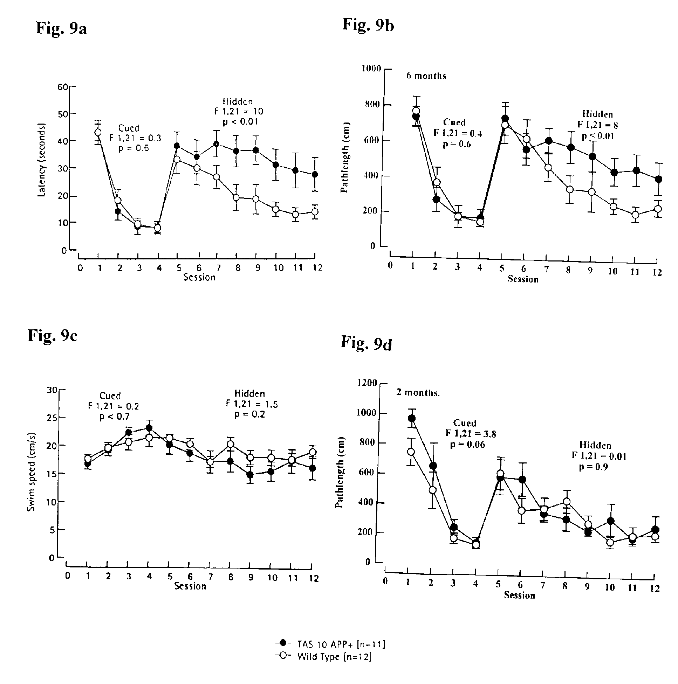

Figure 9 illustrates the results of the Morris Water Maze test. Figure 9a shows latency for 6 month old mice, Figure 9b shows path length for 6 month old mice, Figure 9c shows swim speed for 6 month old mice and Figure 8c shows path length for 2 month old mice to locate the visible platform in the cued task (sessions 1-4) and for locating the hidden platform (sessions 5-12).

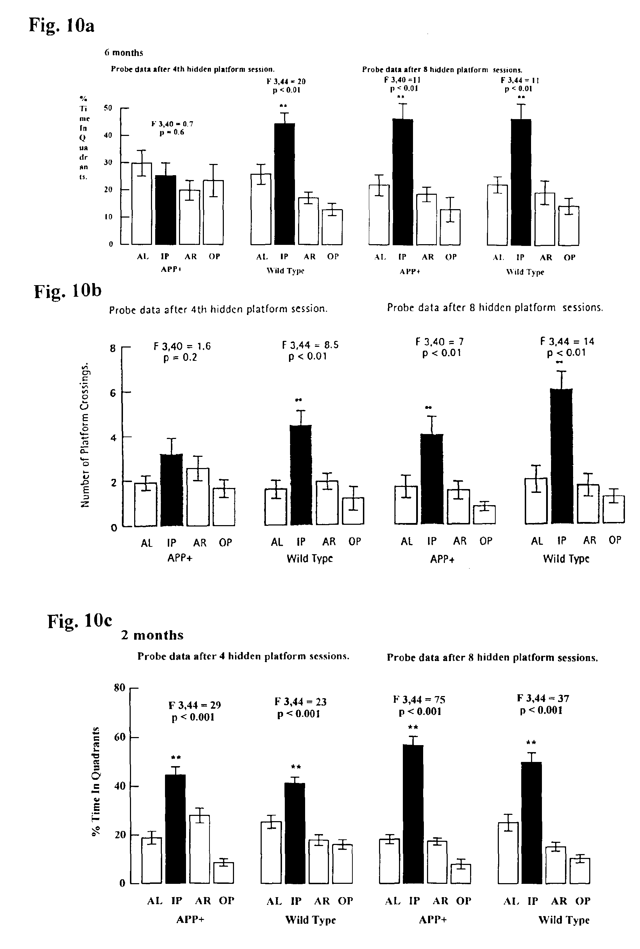

Figure 10 shows the results of a probe trial after the fourth and eighth hidden platform sessions for 6 month old and 2 month old TAS 10 mice. Figure 10a shows the percentage of time spent in each of the quadrants and Figure 10b illustrates the number of platform crossings for 6 month old mice. Figure 10c shows the percentage of time spent in each of the quadrants.

### Detailed Description of Invention

### Constructs

The invention relies on the use of a particular DNA construct to generate a transgenic animal model for screening agents for potential use in the treatment of degenerative brain conditions. The construct consists essentially of a sequence which encodes APP695 containing the Swedish mutation (APP695sw) and a brain-specific Thy-1 regulatory sequence. The brain-specific Thy-1 regulatory sequence may direct expression of the APP695sw selectively in neurons of the brain of the transgenic animal model whilst no expression occurs in the thymus of the animal.

The coding sequence for the human APP695sw may be a cDNA or genomic clone. As noted above, the APP695sw protein comprises two point mutations K670N and M671L as the only mutation in the APP695 polypeptide sequence. Asparagine and leucine residues are therefore present at amino acids 670 and 671 respectively of the APP695sw protein.

The sequence encoding the human APP695 isoform containing the Swedish mutation is operably linked to a brain-specific regulatory region of a Thy-1 gene. Thus the coding sequence is typically linked to a portion of the Thy-1 gene which comprises the regulatory region. The regulatory region does not cause expression of the coding sequence in cells which are not brain cells, such as thymus cells.

The regulatory portion may comprise a Thy-1 gene with one or more deletions. Such a deletion may consist of coding sequence and/or intron sequence. In a preferred embodiment the deletion comprises intron III, such as the deletion shown in Figure 1. Deletion of intron III abolishes the ability of regulatory regions of a Thy-1 gene to direct expression in the thymus. Typically such deletion(s) cause a decrease in the expression of coding sequence from the construct in cells which are not brain cells, such as thymus cells. Typically the deletion(s) does not cause a decrease in expression of coding sequence from the construct in brain cells.

In a preferred embodiment, the regulatory region comprises a Thy-1 gene that has been engineered to drive expression of the coding sequence in brain cells. Such engineered sequences are known in the prior art, for example as described in Vidal *et al* (1990) EMBO J., 9(3), 833-40. The engineered sequences may comprise one or more (e.g. from 2 or 3 up to 5, 10 or 20 or more) insertions, substitutions or deletions (e.g. the deletions described above).

The regulatory region consists of or comprises the Thy-1 promoter. The regulatory region is typically mammalian. It may be human, but is preferably a rodent region, such as a murine region. Typically the region is native to the species of cell in which it is to be expressed. The Thy-1 promoter may be a human promoter but preferably is a rodent Thy-1 promoter such as the murine Thy-1 promoter. The Thy-1 promoter is typically the native promoter for the Thy-1 gene of the animal that is to be generated.

The construct may also comprise, 5' and 3' to the coding sequence, sequences which aid expression, such as aiding transcription and/or translation of the coding sequence. Typically the construct comprises (as well as a Thy-1 promoter) an enhancer, transcription terminator, polyadenylation signal, polyA tail, intron, translation initiation codon and/or translation stop codon.

The construct of the invention may also be used to produce antisense RNA. The antisense polynucleotide may comprise the sequence encoding the APP 695sw protein linked to a Thy-1 promoter in an antisense orientation. Such polynucleotides may be used to produce antisense mRNA to alter the expression of endogenous APP homologues and probe their normal function or may be transfected into brain cells of non-human transgenic animals of the invention to control the levels of transgene expression.

### Vectors

The construct of the invention may be incorporated into a recombinant replicable vector. The vector may be a cloning vector that can be used to replicate the polynucleotide in a compatible host cell, for example a bacterium, or introduce further mutations into the polynucleotide using routine techniques.

The construct of the invention alternatively may be provided in an expression vector containing regulatory sequence(s) to enhance or reduce transcription in addition to the Thy-1 regulatory sequences. The vector may be a viral vector which can be used to transfect efficiently neurones and other cells in the brain.

### Animals

The invention provides non-human transgenic animals comprising a diploid genome containing a coding sequence for the APP695sw protein and a brain-specific Thy-1 regulatory sequence which is operably linked to the coding sequence. Thus the genome of the animal can comprise the construct of the invention. Over-expression of the mutant APP isoform in neurons of the brain of the transgenic animals results in a transgenic animal model that develops the pathology of AD in a time-dependent manner. The non-human transgenic animals develop a cognitive deficit, in particular a spatial memory deficit, that correlates with the accumulation of βA₄ in the brain.

The human APP695sw isoform is typically expressed in brain cells of the animal in an amount from 5 to 12 times greater than the amount of endogenous APP that is expressed in the cells. The brain cells in which over-expression occurs are preferably cells of the hippocampus or cortex. Over-expression does occur in the cerebellum. No expression of the APP695sw protein occurs in the thymus,

The non-human transgenic animals include original founder animals, subsequent progeny that express the transgene any number of generations removed from the founder animals, and crosses of founder animals or their progeny with other animals of the same species. Further non-human transgenic animals can thus be generated by crossing the animals of the invention that over-express human APP695 containing the Swedish mutation with transgenic animals of the same species that express other aspects of AD or genes linked to the disease. One such case is where a human APP695sw transgenic of the current invention is crossed with a transgenic expressing a human Presenilin (PS)-1 cDNA containing the familial mutations (M146V or E280G) under the control of Thy-1 regulatory elements and this develops an Alzheimer-like pathology at earlier ages. Transgenic non-human animals according to the invention can also be provided which express mutant human PS-1, human tau, human apoE2, human apoE3 or human apoE4.

The transgenic animals are generally mammals, especially rodents. Preferred is a transgenic mouse. For mice, a preferred background genetic strain is pure c57bl/6 and a more preferred background genetic strain is c57bl/6 x c57bl/6 xC3H.

### Cells

This invention includes any cells cultured from the transgenic non-human animal. The cells are cultured *in vitro.* The genome of the cells can thus comprise the construct of the invention. The human APP695sw isoform is typically expressed in such cells in an amount from 5 to 12 times greater than the amount of endogenous APP that is expressed in the cells.

Cells cultured *in vitro* from a transgenic animal may be prepared by any suitable method. The cells are typically rodent and preferably mouse cells. Cultures of neuronal cells can therefore be provided, for example cultures of hippocampal or cortical cells. The cells may be used to introduce other genes of interest by any known method (including viral delivery).

The invention also includes any cell transformed or transfected with the polynucleotide of the invention. Such cells include bacterial and yeast cells used to replicate a vector comprising the polynucleotide and stem cells or oocytes that are used to generate the non-human transgenic animals of the invention.

### Production of Animals

The non-human transgenic animals may be generated by the use of any appropriate protocol. A suitable method comprises:
- making a suitable cell of the invention;
- allowing the cell to develop into an animal of the invention; and
- optionally, breeding the animal true.

A construct of the invention may be introduced into an animal embryo by microinjection. Alternatively, embryonic stem cells may be used. Whichever approach is taken, transgenic animals are then generated. The founder animals that are obtained can be bred. The pro-nuclear microinjection route is in fact preferred, in which a construct of the invention is injected into the pronucleus of a fertilised oocyte of a non-human animal.

The transgenic animals can be analysed for the presence of the transgene (i.e. genotyped) in any suitable fashion. Typically, genomic DNA from an animal is screened using PCR with an antisense primer in the APP 695sw coding sequence and a sense primer in the Thy-1 gene into which the coding sequence has been inserted. Results are confirmed by Southern blotting the genomic DNA from the positive samples and probing with a radiolabelled cDNA construct of the invention (i.e. a Thy-1-APP probe).

To obtain transgenic animals that exhibit age-dependent spatial memory deficits that correlate with increasing amyloid burden, it is important to select a line of transgenic animals that exhibits an appropriate level of over-expression of the transgene. Only lines that express the mutant APP transgene at least five times over endogenous APP levels should be selected. Such selected lines can then be subjected to pathological, behavioural and electrophysiological analysis.

Expression levels of the human APP695sw protein relative to the endogenous APP protein can be determined using immunoblotting techniques (typically Western blot analysis). Such levels are generally determined in cells from the hippocampus, cortex or cerebellum of a sacrificed animal. The effects of expression can be analysed by biochemistry, histology and/or immunohistochemistry. Behavioural analysis can be conducted using a cognitive test such as the Y maze test or the Morris Water Maze test.

Expression levels may be determined when a transgenic animal has been obtained, i.e. at time = 0. Expression levels, histology, immunohistochemistry and behavioural analysis can also be determined at time = 3, 6, 9, 12, 18 and/or 24 months. The transgenic animals over-expressing the APP695sw under control of the Thy-1 promoter have been found to develop amyloid pathology. Furthermore, spatial memory deficits have been shown to correlate with increasing amyloid burden, typically at time = 6 months.

### Use of transgenic cells and animals

Cells and animals of this invention may also be used to validate, for example identify and test, candidate secretase(s) or other key factors in the amyloidogenic pathway of the disease. For example, β-secretases and γ-secretases which cleave APP to produce βA₄ can be validated. The cells and animals may be used to investigate the role of APP and βA₄ in Alzheimers disease. For example, antisense RNA may be used to investigate the reversibility of the βA₄ associated pathology.

Cells and animals of this invention may also be used to test the efficacy of potential therapeutic agents for the treatment of conditions characterised by accumulation of βA₄, for example neurodegenerative diseases such as head injury and especially Alzheimers disease. Potential therapeutic agents may inhibit production, deposition or toxicity of βA₄ peptide. A method of identifying a therapeutic agent for the treatment of a condition characterised by accumulation of βA₄ can therefore be provided, comprising:
- administering to an animal of the invention a candidate test substance, and
- determining whether the candidate substance (i) prevents or delays the onset of the condition or (ii) treats the condition.

Option (ii) may be tested by determining whether the candidate substance causes a decrease in any of the cellular or physiological changes caused by the condition, such as those mentioned herein. A method of identifying a therapeutic agent for the treatment of a condition characterised by accumulation of βA₄ can also be provided, comprising:
- contacting a candidate substance with a cell of the invention, and
- determining whether the candidate substance (i) prevents or delays the onset of cellular changes associated with the condition, or (ii) causes a decrease in any of the cellular changes caused by the condition (such as any of the cellular changes mentioned herein).

Option (ii) may be tested, for example, by determining whether the candidate substance inhibits cleavage of mutant APP to βA₄.

Suitable candidate substances which may be tested in the above methods include antibody products (for example, monoclonal and polyclonal antibodies, single chain antibodies, chimeric antibodies and CDR-grafted antibodies). Furthermore, combinatorial libraries, defined chemical identities, peptide and peptide mimetics, oligonucleotides and natural product libraries, such as display libraries (e.g. phage display libraries) may also be tested. The candidate substances may be chemical compounds. Batches of the candidate substances may be used in an initial screen of, for example, ten substances per reaction, and the substances of batches which show inhibition tested individually.

The transgenic animal models of the invention may also be used to test the efficacy of potential therapeutic agents aimed at inhibiting downstream events of APP processing, eg. phosphorylation of tau, neuronal apoptosis and inflammation. One example of testing therapeutic agents involves incubation of the recombinant cells and brain tissue of the invention with antisera recognising βA₄ amyloid, which is the major characteristic of senile and diffuse amyloid plaques. Testing for agents that affect the level of tau phosphorylation and tangle formation, state of microglial activation and extent of neuronal loss is particularly preferred for transgenic animals, and cells from such animals that contain a mutation, such as a mutation in a Presenilin gene in addition to the APP transgene of the invention.

Agents which alter βA₄ production and/or which alter the AD like pathology can therefore be tested *in vivo* or in cells or tissue preparations *in vitro.* Compounds can be tested using the assays and tests used to characterise the invention. For example, after administration of any potential therapeutic agent, the response of the transgenic animal may be assessed by behavioural and electrophysiological tests as described in the Examples. Methods for screening potential therapeutic agents using cell lines or animals are established. ELISA or Homogenous Time Resolved Fluorescence (HTRF) methodologies can be used.

Agents identified in the screening methods of the invention may be used to prevent or treat the diseases discussed above. The condition of a patient suffering from such a disease can therefore be improved by administration of such a product. A therapeutically effective non-toxic amount of such an agent may be given to a human patient in need thereof.

The formulation of the product for use in preventing or treating the disease will depend upon factors such as the nature of the agent identified and the disease to be prevented or treated. Typically the agent is formulated for use with a pharmaceutically acceptable carrier or diluent. For example it may be formulated for intracranial, parenteral, intravenous, intramuscular, subcutaneous, transdermal or oral administration. A physician will be able to determine the required route of administration for each particular patient. The pharmaceutical carrier or diluent may be, for example, an isotonic solution.

The dose of product may be determined according to various parameters, especially according to the substance used; the age, weight and condition of the patient to be treated; the route of administration; and the required regimen. A suitable dose may however be from 0.1 to 100 mg/kg body weight such as 1 to 40 mg/kg body weight. Again, a physician will be able to determine the required route of administration and dosage for any particular patient.

The construct, cells or therapeutic agents of the invention may be present in a substantially isolated form. It will be understood that the they may be mixed with carriers or diluents which will not interfere with their intended purpose and still be regarded as substantially isolated. Thus the construct, cell or therapeutic agent of the invention may also be in a substantially purified form, in which case it will generally comprise more than 90%, e.g. more 95%, 98% or 99%, by weight of the relevant preparation.

The following Examples illustrate the invention

### Example 1: Transgene construction

A Thy-1-APP transgene was generated by inserting the 695 isoform of human APP cDNA (APP695) harbouring the Swedish familial mutation into a vector containing the murine Thy-1 gene. This insertion replaced the coding sequence of the Thy-1 gene allowing brain-specific expression of the transgene (Figure 1). The 5' end of the APP coding sequence was preceded by a blunted HindIII site and a strong Kozak translation initiation sequence. All modifications were made using standard cloning methods and PCR-based site-directed mutagenesis.

### Example 2: Generation of transgenic mice and screening of founders

The Thy-1-APP gene fusion DNA was dissolved in TE (10mM Tris, 1mM EDTA, pH 8) to a final concentration of 10µg/ml prior to injection. Transgenic lines were generated by pro-nuclear microinjection of this fragment, after removal of backbone plasmid sequence, into fertilised oocytes from c57bl/6xC3H mice.

Tail biopsy DNA was generated by standard methods and 1µl (of 50µl) was used in a PCR reaction with a 5' Thy-1 primer and a 3' APP primer. Transgene copy was determined by Southern blotting using 10µg of denatured purified tail DNA and a radiolabelled 0.7kb BamHI fragment.

### Example 3: Analysis of Transgene Expression

APP transgene products were examined in progeny of transgenic founders sacrificed at 1-4 months of age. Protein extracts from brains of transgenic and littermate controls were prepared by homogenisation of half-brains in RIPA lysis buffer (50mM Tris pH 8, 150mM NaCl, 1% sodium deoxycholate, 1% Triton-X, protease inhibitors) followed by centrifugation at 13000 rpm for 20 minutes at 4°C (Moechars *et al.,* EMBO J. 15(6), 1265-1274, 1996). SDS was added to the supernatants to a final concentration of 1% and equal amounts (20µg) of protein were fractionated using 8% SDS-PAGE. Expression of the APP transgene from nine TAS lines (44,138, 36,102,10, 74, 25, 39 and 99) was determined using the LN27 monoclonal anti-APP antibody (Zymed). The data are shown in Table 1 below.

**Table 1:**

| Expression of APP transgene | | |
|---|---|---|
| Line | Approx. copy number. | Expression (x over endogenous) |
| TAS10 | 40 | 12x |
| TAS25 | 25 | 12x |
| TAS36 | 2 | 3x |
| TAS39 | 5 | 2x |
| TAS44 | 5 | 2x |
| TAS74 | 50 | 10x |
| TAS99 | 3 | 2x |
| TAS102 | 25 | 7x |
| TAS138 | 3 | 4x |

The four highest expressing lines (TAS 10, 25, 74 and 102) were crossed with c57bl/6 mice to generate large numbers of F2 mice on a c57bl/6/C3Hxc57bl/6 genetic background for use in this longitudinal study. Expression of the transgene was confirmed to be brain-specific and enriched in the hippocampus and cortex relative to the cerebellum.

### Example 4: Analysis of βA₄ in Brain Tissue

Brains from six non-transgenic control mice and six transgenic mice from the four highest expressing TAS lines were analysed for levels of total βA₄. Brains were removed and immediately hemisected; half was fixed in 2% paraformaldehyde for histological analysis and half was dissected into hippocampal, cortical and cerebellar tissue. Accurate mass measurements of the tissue pieces were recorded, before lysing tissue samples using guanidine/casein extraction buffer (Johnson-Wood *et al.,* PNAS USA 94(4), 1550-1555, 1997).

Sandwich ELISAs (Suzuki *et al.,* Science 264, 1336-1340, 1994) were carried out using 4G8 capture and biotinylated 6E10 reporter antibodies (Kim *et al.* Neurosci. Res. Comm. 7(2), 113-130, 1990; Figure 2). Samples were tested in triplicate and absorbances which fell within the range of a βA₄ 1-40 standard curve (1-20ng/ml) were converted to picomoles and expressed as mean pmol/g wet tissue for each TAS line, either non-transgenic or transgenic.

Mean βA₄ values (± SEM) for each non-transgenic and transgenic TAS line are displayed in Figures 3, 4 and 5 for hippocampal, cortical and cerebellar tissues respectively from mice sacrificed at 6 months. Values are in pmol/g wet tissue.

Mean βA₄ values (± SEM) in brain samples from non-transgenic (Fig. 6a) and transgenic mice (Fig. 6b) from the TAS 10 line sacrificed at 2, 6, 12, 18 and 24 months are shown for hippocampal, cortical and cerebellar tissues. The βA₄ load in all three brain regions of the littermate wild type mice is equivalent to background levels throughout the longitudinal study.

Sandwich ELISAs were also employed to measure levels of 1-40 and 1-42 βA₄ as described above, but using antibodies raised against the peptide CMVGGVV to capture the 1-40 species of βA₄ and antibodies raised against the peptide CMVGGVVIA to capture the 1-42 βA₄ species. Mean 1-40 βA₄ and 1-42 βA₄ values (± SEM) in brain samples from non-transgenic (Fig. 7a) and transgenic mice (Fig. 7b) from the TAS10 line sacrificed at 2, 6, 12, 18 and 24 months are shown for hippocampal, cortical and cerebella tissues.

Immunohistological analysis was carried out after brains were fixed in paraformaldehyde. Sections of brain through the hippocampus and cortex were stained with a range of antisera raised against βA₄. Amyloid plaque pathology was detected in the cortex of brains from transgenic mice from TAS lines sacrificed at 12 months. For this purpose, three antisera were used that recognised amino acids 1-17, 35-40 and 35-42 respectively of βA₄.

Immunohistochemical analysis using end-specific antisera against βA₄ 1-40 and 1-42 was also carried out to detect age dependent deposition of β-amyloid in the brains of transgenic mice sacrified at 12, 18 and 24 months.

At 18 months mean plaque size, number and proportion of anatomically defined region occupied by plaques in the hippocampus of TAS 10 mice is significantly greater than at 12 months. The results of image analysis of the plaque load in the hippocampus of TAS 10 mice at 18 months of age are shown in Figure 8. The amyloid burden is expressed as % of the total sample area in each of the dentate gyrus (DG), CA1 and CA3 regions occupied by plaques.

Counter-staining for Thioflavin S revealed that fibrillar plaques are much more common in 18-month old transgenics than in 12 month old transgenics. Age-matched wild-type littermate controls lack amyloid plaques in all regions studied.

Evidence of cell loss was evident in the hippocampus of the TAS10 transgenic mouse brain at 24 months in the region surrounding Thioflavin S positive plaques. Highly pigmented, condensed cells were also visible in this region suggestive of cells undergoing programmed cell death or the presence of inflammatory cells such as microglia.

Staining with antibodies to glial fibrillary acidic protein (GFAP) (Sigma, G9269) showed that activated astrocytes co-localised with large cortical amyloid deposits and that activated astrocytes were present throughout the hippocampus.

### Example 5: Neurological Assessments and Locomotor Activity

Test groups were selected that comprised 12 male TAS mice and wild-type littermate controls. All mice were subjected to a battery of tests to probe spinal cord (flexion), medulla (corneal reflex), pons (righting reflex), and autonomic (salivation) functions. Motor co-ordination was assessed on a mouse rota-rod and forelimb grip strength was determined with a grid floor and pressure transducer. Mice were individually placed in activity monitor cages and locomotor activity recorded for 1 hour. There was no prior habituation. Locomotor activity (LMA)was quantified by measuring the number of beam breaks.

Table 2 shows the results of a neurological assessment exemplified for TAS 10 mice in comparison with wild-type controls at 2, 6, 12, 18 and 24 months. No significant difference was noted in the sensory/motor tasks at any age.

**Table 2:**

| Neurological Assessment of TAS10 APP over-expressing mice and wild-type controls | | | | | | | |
|---|---|---|---|---|---|---|---|
| Age | n | Genotype | LMA | RotaRod | Grip Strent. (g) | Body Temp (°C) | Body Weight (g) |
| TAS 10 2 months | 11 | APP+ | 3132±463 | 161±14 | 89±2.4 | 37.3±0.1 | 23.6±0.7 |
| | 12 | WT | 3003±324 (p=0.9) | 166±15 (p=0.8) | 81±5 (p=0.1) | 37.3±0.1 (p=0.6) | 25±0.7 (p=0.1) |
| TAS 10 6 months | 12 | APP+ | 1375±244 | 82.1±8 | 72.3±6 | 37.5±0.2 | 30.3±1 |
| | 12 | WT | 1452±110 (p=0.8) | 103±12 (p=0.2) | 82±4 (p=0.2) | 37.4±0.1 (p=0.7) | 36.8±1.3** (p<0.001) |
| TAS 10 12 months | 12 | APP+ | 2878±352 | 64±10 | 86±7 | 37±0.2 | 36.7±2 |
| | 12 | WT | 2698±343 (p=0.7) | 48±7 (p=0.2) | 92±7 (p=0.5) | 37.2±0.1 (p=0.5) | 40±1.2 (p=0.1) |
| TAS 10 18 months | 12 | APP+ | 2619±34 | 66±10 | 59±4 | 37.8±0.1 | 24.3±0.4 |
| | 12 | WT | 2579±43 (p=0.9) | 64±9 (p=0.8) | 52±4 (p=0.1) | 38±0.1 (p=0.7) | 25.6±0.6 (p=0.1) |
| TAS 10 24 months | 12 | APP+ | 2789 | 59±15 | 75±5 | 37.1±0.1 | 38.4±2 |
| | 12 | WT | 2362 (p<0.01) | 59±13 (p=0.9) | 68±7 (p=0.5) | 37±0.1 (p=0.7) | 40.6±3 (p=0.5) |

### Example 6: Cognitive test : the Morris Water Maze Test

At 2 and 6 months of age mice were trained over 4 sessions using the cued escape platform (cued task). Each trial was commenced by placing the mouse gently in the pool and activating an overhead camera connected to a computerised tracking system that recorded the swim paths of the mice to locate the platform. Following the cued task, the mice received 8 sessions with the hidden platform to assess spatial reference learning. After the 12 sessions the mice underwent a probe trial which consisted of a 60 second trial without the platform present during which the search pattern was analysed. The time spent searching each quadrant was recorded as a percentage of the total time. The time spent searching the quadrant that formerly contained the hidden platform was compared to that spent searching in the other quadrants.

Figure 9 shows the results of the Morris Water Maze test. Figures 9a shows latency, Figure 9b shows path length and Figure 9c shows swim speed for locating the visible platform in the cued task (sessions 1-4) and for locating the hidden platform (sessions 5-12) for 6 month old mice. Figure 9d shows path length for locating the visible platform (sessions 1-4) and for locating the hidden platform (sessions 5-12) for 2 month old mice. There was no main effect of genotype in the cued task at 2 or 6 months. However, 6 month old mice but not 2 month old mice from the TAS 10 transgenic line did show a main effect of genotype (F, 1,21 = 10; p = 0.01) when locating the hidden platform which suggests a deficit in spatial learning. Similarly, the probe trial showed evidence of a spatial memory impairment in this transgenic line at 6 months (Figures 10a and 10b) but not at 2 months (Figure 10c).

### Example 7: Cognitive test: y-MAZE Spontaneous Alternation

The maze consisted of three wooden arms 50cm long, 30cm high, and 12cm wide that converged at an equal angle. The arms were arbitrarily assigned A, B and C. Each mouse was placed randomly at the end of one of the arms and the number and sequence of arm entries recorded manually for five minutes. The Y-maze exploits the natural tendency of the mice to explore a new environment and alternation describes entry into all three arms on consecutive choices. The maximum number of alternation opportunities equalled the total number of arm entries minus two and percent alternation was calculated as actual alternations divided by maximum alternation opportunities x 100.

Analysis by unpaired *t*-test showed no evidence for a genotype specific difference in spontaneous alternation, position bias or total arm entries at 2 or 6 months of age (Table 3). However, at 12 and 18 months there was a significant and progressive impairment of spontaneous alternation behaviour indicating a working memory deficit in the APP over-expressing mice. There was no significant difference in position bias or arm entries between the groups (Table 3).

**Table 3:**

| Performance of TAS 10 transgenic mice and wild type controls in the Y-maze. | | | | | |
|---|---|---|---|---|---|
| | Age | n | % Alternation | Arm Entries | Position bias |
| Wild Type | | 12 | 64±5 | 22± | 47 |
| | 2 months | | | | |
| APP | | 12 | 72±5 | 22 | 37 |
| Wild Type | | 12 | 63±5 | 20±2 | 48±6 |
| | 6 months | | | | |
| APP | | 12 | 65±5 | 20±1 | 48±4 |
| Wild Type | | 12 | 72±4 | 23±2 | 23±2 |
| | 12 months | | | | |
| APP | | 12 | 62±6* | 25±3 | 25±3 |
| Wild Type | | 12 | 71±5 | 23±2 | 45±7 |
| | 18 months | | | | |
| APP | | 12 | 57±5** | 21±2 | 36±4 |
| Wild Type | | 12 | 62±7 | 21±2 | 41±7 |
| | 24 months | | | | |
| APP | | 12 | 58±3 | 22±1 | 52±4 |

### Example 8: Contextual and Cued Fear Conditioning

The mice were initially allowed to freely explore the training chamber for 2 minutes in the presence of white noise to provide background sound. Following the 2 minutes exploration time the mice were presented with a 20 second 80dB tone along with a 24 W intra-chamber light that co-terminated with a 1 second foot-shock (0.5mA). The 1 sec foot-shock was followed by two more tone-shock pairings at 2 minute intervals. Contextual memory was measured by determining the number of second freezing exhibited by mice in the presence of the old context (without light/tone shock presentations). Cued memory was determined 2 hours later in the same mice in a new environment (different colour, texture). The mice were placed in the novel context for 1 minute after which a 20 second 80 dB tone was presented along with the light. This was repeated twice and the mean of the 2 one minute data sets was calculated.

There were no significant difference in contextual or cued memory between wild type or APP over-expressing mice at any of the age points studied.

## Claims

1. A recombinant DNA construct comprising a coding sequence which encodes the human APP695 isoform containing only the mutations K670N and M671L and a brain-specific Thy-1 regulatory sequence which is operably linked to the coding sequence.

2. A construct according to claim 1 wherein the regulatory sequence includes a rodent Thy-1 promoter.

3. A construct according to claim 2 wherein the Thy-1 promoter is the murine Thy-1 promoter.

4. A transgenic non-human animal whose genome incorporates DNA comprising a coding sequence which encodes the human APP695 isoform containing only the mutations K670N and M671L and a brain-specific Thy-1 regulatory sequence which is operably linked to the coding sequence, the said mutated human APP695 isoform being expressed in brain cells of the animal in an amount at least five times greater than the amount of endogenous APP that is expressed therein.

5. A transgenic non-human animal wherein the said brain cells are cells of the hippocampus, cerebellum or cortex.

6. A transgenic non-human animal according to claim 4 or 5 which is a rodent.

7. A transgenic non-human animal according to claim 6 which is a mouse.

8. A transgenic non-human animal according to any one of the claims 4 to 7 which also expresses mutant human PS-1, human tau, human apoE2, human apoE3 or human apoE4.

9. A transgenic non-human animal cell whose genome incorporates DNA comprising a coding sequence which encodes the human APP695 isoform containing only the mutations K670N and M671L and a brain-specific Thy-1 regulatory sequence which is operably linked to the coding sequence, the said mutated human APP695 isoform being expressed in the said cell in an amount at least five times greater than the amount of endogenous APP that is expressed therein.

10. A cell according to claim 9 which is a rodent cell.

11. A cell according to claim 10 which is a mouse cell.

12. A cell according to any one of claims 9 to 11 which also over-expresses mutant human PS-1, human tau, human apoE2, human apoE3 or human apoE4.

13. A method of producing a transgenic non-human animal as defined in claim 4, which method comprises:
(a) introducing a construct as defined in any one of claims 1 to 3 into a cell or embryo; and
(b) generating a said animal from said cell or embryo.

14. A method according to claim 13 wherein the construct is injected into the pronucleus of a fertilized oocyte of a non-human animal.

15. A method according to claim 13 or 14 wherein the founder animals that are obtained are bred.

16. A method of screening agents for use in the treatment of a degenerative brain condition, which method comprises screening the said agents using a transgenic non-human animal as defined in any one of claims 4 to 8 or a transgenic non-human animal cell as defined in any one of claims 9 to 12.

17. A method according to claim 16 wherein the degenerative brain condition in Alzheimer's disease.

18. A kit for screening agents for use in the treatment of a degenerative brain condition, which kit comprises a transgenic non-human animal cell as defined in any one of claims 9 to 12 and a means for determining whether a test agent inhibits cellular changes associated with mutated APP expression in said cell.

19. An agent identified using a method according to 16 or 17 or a kit according to claim 18.

20. An agent according to claim 19 for use in the treatment of a degenerative brain condition.

21. Use of an agent according to claim 20 in the manufacture of a medicament for the treatment of Alzheimer's disease.

22. A pharmaceutical composition suitable for use in the treatment of a degenerative brain condition, which composition comprises a pharmaceutically acceptable carrier or diluent and, as active ingredient, an agent identified using a method according to claim 16 or 17 or a kit according to claim 18.

23. A transgenic non-human animal that exhibits increasing amyloid burden and associated correlative cognitive decline in an age-related manner.
